# EUROPÄISCHE PATENTANMELDUNG

(11) **EP 3 569 286 A1**
(43) Veröffentlichungstag der Anmeldung: **20.11.2019**
(21) Anmeldenummer: 19175101.5
(22) Anmeldetag: 17.05.2019
(51) Int. Cl.: A61N 2/02, A61N 2/00, A61N 5/06

(54) **WELLNESSVORRICHTUNG**

(30) Priorität: 17.05.2018 DE 202018002413 U
(71) Anmelder: Schieber, Gerhard, 70825 Korntal-Münchingen (DE); Müller, Uwe, 24782 Büdelsdorf (DE); Kienle, Thomas, 70499 Stuttgart (DE)
(72) Erfinder: Schieber, Gerhard, 70825 Korntal-Münchingen (DE); Müller, Uwe, 24782 Büdelsdorf (DE); Kienle, Thomas, 70499 Stuttgart (DE)
(74) Vertreter: Wasmuth, Rolf

(57) **Zusammenfassung**

Eine Wellnessvorrichtung umfasst ein Gehäuse (2), wobei das Gehäuse (2) einen sich in Längsrichtung des Gehäuses (2) erstreckenden Behandlungsraum (5) umschließt. Die Wellnessvorrichtung (1) umfasst eine zentrale Steuereinheit (18) zur Steuerung der Wellnessvorrichtung (1). An dem Gehäuse sind mindestens ein Infrarotstrahler (3) zur Erzeugung von Infrarotstrahlung im Behandlungsraum (5) und eine Magnetfeldeinheit (4) zur Erzeugung eines im Wesentlichen homogenen Magnetfeldes im Behandlungsraum (5) angeordnet.

## Beschreibung

Die Erfindung betrifft eine Wellnessvorrichtung der im Oberbegriff des Anspruchs 1 angegebenen Gattung. Weiter betrifft die Erfindung ein Verfahren mit einer Wellnessvorrichtung.

Der Erfindung liegt die Aufgabe zugrunde, eine Wellnessvorrichtung zu bilden, welche eine Steigerung des Wohlbefindens des Benutzers ermöglicht.

Die Aufgabe wird durch eine Wellnessvorrichtung mit den Merkmalen des Anspruchs 1 gelöst.

Zudem liegt der Erfindung die Aufgabe zugrunde, ein Verfahren zu bilden, welches die Steigerung des Wohlbefindens des Benutzers ermöglicht.

Die Aufgabe wird durch ein Verfahren mit einer Wellnessvorrichtung mit den Merkmalen des Anspruchs 13 gelöst.

Die Wellnessvorrichtung umfasst ein Gehäuse, wobei das Gehäuse einen sich in Längsrichtung des Gehäuses erstreckenden Behandlungsraum umschließt. Zudem umfasst die Wellnessvorrichtung eine zentrale Steuereinheit zur Steuerung der Wellnessvorrichtung. An dem Gehäuse ist mindestens ein Infrarotstrahler zur Erzeugung von Infrarotstrahlung im Behandlungsraum angeordnet. Zudem ist an dem Gehäuse eine Magnetfeldeinheit zur Erzeugung eines im Wesentlichen homogenen Magnetfeldes im Behandlungsraum angeordnet. Das erzeugte Magnetfeld ist vorzugsweise gepulst.

Es hat sich gezeigt, dass die zeitgleiche Erzeugung von Infrarotstrahlung und eines im Wesentlichen homogenen Magnetfeldes im Behandlungsraum des Gehäuses auf einen Benutzer, welcher sich im Behandlungsraum befindet, zu dessen Wohlbefinden beiträgt.

Die Magnetfeldeinheit umfasst Magnetspulen, wobei die Magnetspulen vorzugsweise in einer Helmholtzanordnung angeordnet sind. Die Magnetspulen sind auf derselben Achse parallel aufgestellt und gleichsinnig vom Strom durchflossen. Das Feld einer jeden einzelnen Magnetspule ist grundsätzlich inhomogen. Durch die Überlagerung beider Felder ergibt sich zwischen beiden Spulen nahe der Spulenachse ein Bereich mit einem im Wesentlichen homogenen Magnetfeld.

Die Magnetspulen sind vorteilhaft ringartig ausgebildet und umschließen den Behandlungsraum. Die Größe des Behandlungsraumes ist folglich durch den Spulendurchmesser begrenzt. Der Spulendurchmesser der Magnetspulen ist dabei derart groß ausgelegt, dass einem Benutzer ausreichend Raum im Behandlungsraum zur Verfügung steht. Der Durchmesser einer Magnetspule ist vorzugsweise mindestens 80 cm, vorteilhaft mindestens 100 cm, insbesondere mindestens 120 cm.

Es ist vorteilhaft vorgesehen, dass dem Infrarotstrahler ein B/C-Absorber zur Filterung von mittel- und langwelliger Infrarotstrahlung nachgeschaltet ist. Der B/C-Absorber ist vorzugsweise separat von dem mindestens einen Infrarotstrahler ausgebildet. Der B/C-Absorber umfasst zwei zueinander parallel angeordnete Glasplatten, wobei die Glasplatten einen Hohlraum einschließen und der Hohlraum mit einer Salzwasserlösung gefüllt ist. Der Infrarotstrahler emittiert A/B/C-Strahlung. Die mittel- und langwellige Infrarotstrahlung kann auf der Haut des Benutzers durch Absorption von B/C-Infrarotstrahlung eine besonders hohe Hitze auf der Haut bilden. Um ein besonders hohes Wohlempfinden des Benutzers zu ermöglichen, ist die B/C-Infrarotstrahlung zu filtern. Hierfür ist der B/C-Absorber vorgesehen. Die A/B/C-Strahlung passiert den B/C-Absorber, welcher aus drei Reflexionszonen, nämlich Glasplatte, Salzwasserlösung und Glasplatte gebildet ist. Die Infrarotstrahlung passiert die erste Glasplatte und dringt in das Salzwasser ein. In dem Salzwasser werden die B/C-Infrarotstrahlungen absorbiert. Lediglich die A-Infrarotstrahlung durchdringt das Salzwasser und anschließend die zweite Glasplatte. Dabei können Transmissionsverluste von in etwa 10% auftreten. Der Abstand der Glasplatten beträgt vorzugsweise mindestens 1 mm. So kann eine ausreichende Absorption der B/C-Infrarotstrahlung gewährleistet werden.

Der mindestens eine Infrarotstrahler ist um eine erste Schwenkachse schwenkbar. Der mindestens eine Infrarotstrahler ist vorteilhaft um eine zweite Schwenkachse schwenkbar. Es ist vorgesehen, dass der mindestens eine Infrarotstrahler zum Schwenken durch einen über die zentrale Steuereinheit gesteuerten Motor für jede Schwenkachse angetrieben ist. Die Infrarotstrahler werden während der Benutzung der Wellnessvorrichtung permanent bewegt, wodurch ein zu langes lokales Wirken auf eine Hautstelle des Benutzers vermieden werden kann. Dadurch kann das Wohlempfinden bei Benutzung der Wellnessvorrichtung erhöht werden.

Es ist vorteilhaft vorgesehen, dass zwei zueinander benachbarte Magnetspulen einen funktionalen Zwischenraum begrenzen, wobei der mindestens eine Infrarotstrahler in dem funktionalen Zwischenraum angeordnet ist. Dadurch kann ein kompakter Aufbau der Wellnessvorrichtung gewährleistet werden.

Die Wellnessvorrichtung umfasst vorzugsweise mindestens einen UVC-Strahler zur Desinfektion des Behandlungsraumes. Nach Benutzung der Wellnessvorrichtung kann mittels der UVC-Strahler der Behandlungsraum desinfiziert werden. Dadurch gestaltet sich für Bediener der Wellnessvorrichtung die Reinigung dieser einfach und zeitsparend.

Das Verfahren mit der erfindungsgemäßen Wellnessvorrichtung umfasst die folgenden Schritte:
Zeitgleiche Erzeugung von Infrarotstrahlung und eines im Wesentlichen homogenen Magnetfeldes im Behandlungsraum des Gehäuses. Der Benutzer wird im Behandlungsraum vorzugsweise auf eine maximale Temperatur von höchstens 38, 5°C erwärmt.

Ausführungsbeispiele der Erfindung sind nachstehend anhand der Zeichnung näher beschrieben. Es zeigen:
- Fig. 1: in einer schematischen Vorderansicht die erfindungsgemäße Wellnessvorrichtung,
- Fig. 2: in einer schematischen Seitendarstellung in Richtung des Pfeils II nach Fig. 1 die erfindungsgemäße Wellnessvorrichtung,
- Fig. 3: in einer schematischen Draufsicht in Richtung des Pfeils III nach Fig. 1 die erfindungsgemäße Wellnessvorrichtung,
- Fig. 4: in einer schematischen Draufsicht eine Infrarotstrahler-Einheit,
- Fig. 5: in einer schematischen Vorderansicht eine alternative Ausführung der erfindungsgemäßen Wellnessvorrichtung,
- Fig. 6: in einer schematischen Vorderansicht eine Höhenverstellung der Infrarotstrahler-Einheit,
- Fig. 7: in einer schematischen Draufsicht die Verstelleinheit der Infrarotstrahler-Einheit,
- Fig. 8: in einer schematischen Seitenansicht die Wellnessvorrichtung nach Fig. 5 und
- Fig. 9: in einer schematischen Vorderansicht die Wellnessvorrichtung nach Fig. 5.

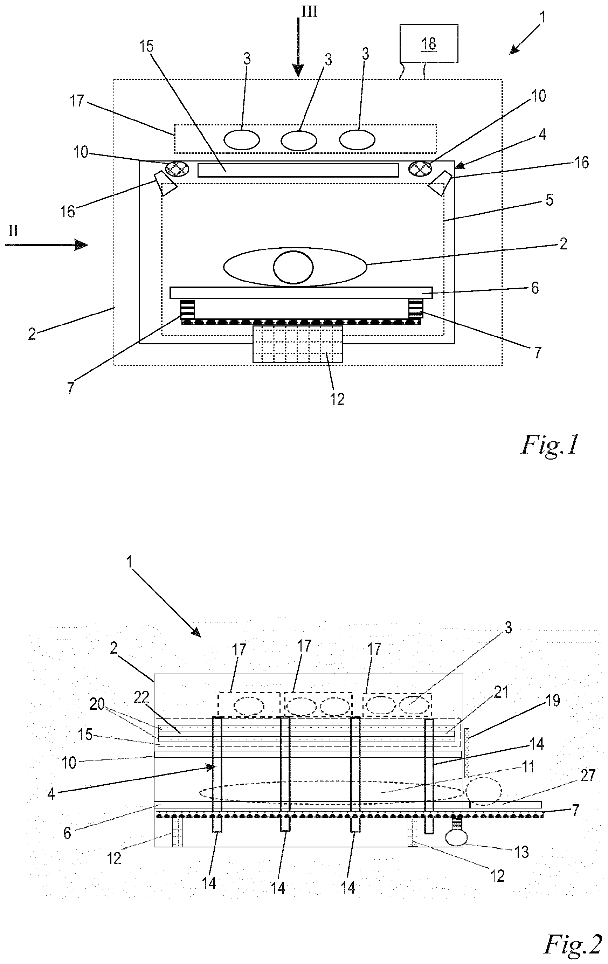

Fig. 1 zeigt die erfindungsgemäße Wellnessvorrichtung. Die Wellnessvorrichtung 1 umfasst ein Gehäuse 2. An dem Gehäuse 2 sind mindestens ein Infrarotstrahler 3 und eine Magnetfeldeinheit 4 angeordnet. Das Gehäuse 2 umschließt einen Behandlungsraum 5, wobei sich der Behandlungsraum 5 in Längsrichtung des Gehäuses 2 erstreckt. Die Wellnessvorrichtung 1 umfasst ferner eine Liegefläche 6, welche im Behandlungsraum 5 angeordnet ist. Bei Gebrauch der Wellnessvorrichtung 1 kann sich der Benutzer 11 auf der Liegefläche 6 positionieren. Die Liegefläche 6 ist auf Teleskopschienen 7 angeordnet. Zwischen dem Behandlungsraum 5 und dem Infrarotstrahler 3 ist ein B/C-Absorber 15 angeordnet. Der B/C-Absorber dient zur Filterung von mittel- und langwelliger Infrarotstrahlung und ist dem Infrarotstrahler 3 nachgeschaltet. Mittels der Anordnung eines Infrarotstrahlers 3 und einer Magnetfeldeinheit 4 an dem Gehäuse 2 ist eine Erzeugung von Infrarotstrahlung und eines im Wesentlichen homogenen, gepulsten Magnetfeldes im Behandlungsraum 5 möglich.

In Fig. 2 ist die Seitenansicht der Wellnessvorrichtung 1 gezeigt. Die Liegefläche 6 ist auf zwei Teleskopschienen 7 angeordnet. Dadurch kann die Liegefläche 6 auf einfache Weise aus dem Gehäuse 2 herausgezogen und wieder hineingeschoben werden. Die Liegefläche 6 ist über zwei Stützräder 13 abgestützt. Dies erleichtert dem Benutzer 11 den Einstieg in die Wellnessvorrichtung 1. Der Benutzer 11 legt sich auf die Liegefläche 6, wird über den Bediener in das Gehäuse 2 hineingeschoben und nach vollendeter Behandlung wieder herausgezogen. Die Liegefläche 6 kann alternativ auch motorisiert bewegt werden. Die Liegefläche 6 ist für eine Tragmasse von mindestens 200 kg ausgelegt. Ferner ist auch alternativ ein nicht dargestellter Klappmechanismus mit einem Deckel des Gehäuses 1 zum Einstieg des Benutzers möglich. Ist der Deckel des Gehäuses 2 aufgeklappt, kann der Benutzer 11 sich auf die Liegefläche 6 legen und anschließend der Deckel geschlossen werden. Wie in Fig. 1 und 2 gezeigt, ist die Liegefläche 6 auf einer Hubverstellvorrichtung 12 befestigt. Über die Hubverstellvorrichtung 12 kann die Liegefläche 6 in ihrer Höhe eingestellt werden und dadurch der Abstand zwischen dem Benutzer 11 und dem Infrarotstrahler 3 angepasst werden.

In der Fig. 2 ist der B/C-Absorber gezeigt, welcher zwei zueinander parallel angeordnete Glasplatten 20 umfasst. Die Glasplatten 20 sind in einem Abstand von mindestens 1 mm, vorzugsweise von in etwa 4 mm zueinander angeordnet. Der von den Glasplatten 4 eingeschlossene Hohlraum 21 ist mit einer partikelfreien Salzwasserlösung 22 befüllt. In Betrieb der Wellnessvorrichtung 1 durchdringt die Infrarotstrahlung zuerst die obere Glasplatte 20 und dringt anschließend in die Salzwasserlösung 22 ein. In der Salzwasserlösung 22 wird die B/C-Infrarotstrahlung absorbiert. Lediglich die A-Infrarotstrahlung durchdringt die Salzwasserlösung und anschließend die zweite Glasplatte 20. So durchdringt die A-Infrarotstrahlung den B/C-Absorber und gelangt in den Behandlungsraum 5, wobei lediglich Transmissionsverluste von in etwa 10% auftreten. Die Lichtbrechung in der Salzwasserlösung ist different, wobei es zu einer Schlierenbildung in der Salzwasserlösung kommt. Folglich durchdringt die A-Infrarotstrahlung den B/C-Absorber mit ungleichmäßigem Durchbrechungswinkel, wodurch eine zu lange gleichmäßige Bestrahlung einer lokalen Hautzone vermieden werden kann. Ferner ist ein Ausgleichsgefäß an den Hohlraum 21 gekoppelt, welches bei Erhitzung der Salzwasserlösung 22 durch die Infrarotstrahler 3 die sich durch die Hitze ausdehnende Flüssigkeit aufnimmt. Die Glasplatten 20 des B/C-Absorbers bestehen aus einem mindestens bis 1100°C hitzeresistenten Glas. Die Glasplatten 20 sind vorzugsweise bruchsicher. Die Glasplatten 20 weisen eine Länge von in etwa 160 cm, eine Breite von in etwa 70 cm und eine Dicke von in etwa 4 mm auf. Damit die Augen des Benutzers 11 gegenüber der Infrarotstrahlung geschützt sind, ist eine Sichtblende 19 vorgesehen, die an dem einen offenen Ende des Gehäuses 2 der Wellnessvorrichtung 1, über welches die Liegefläche 6 eingeschoben wird, angeordnet ist. Der Benutzer 11 ist derart auf der Liegefläche 6 zu positionieren, dass der Kopf des Benutzers 11 außerhalb des Gehäuses 2 angeordnet ist und die Sichtblende 19 den Kopf des Benutzers 11, insbesondere dessen Augen, vor Infrarotstrahlung schützt.

Wie in Fig. 2 gezeigt, umfasst die Wellnessvorrichtung 1 eine Magnetfeldeinheit 4, welche aus vier Magnetspulen 14 gebildet ist. Die Magnetspulen 14 sind in einer Helmholtzanordnung zueinander angeordnet. Jede der vier Magnetspulen 14 ist ringartig ausgebildet und umschließt den Behandlungsraum 5. Der Durchmesser einer Magnetspule 14 beträgt vorzugsweise mindestens 80 cm, insbesondere mindestens 100 cm, in etwa 120 cm. Die Magnetspulen 14 sind zueinander in etwa parallel und zugleich koaxial angeordnet. Benachbarte Magnetspulen 14 weisen einen Abstand von in etwa 40 cm auf. Durch die Helmholtzanordnung können die inhomogenen Magnetfelder einer einzelnen Magnetspule 14 zu einem homogenen Magnetfeld gebündelt werden. Dabei ist die Stromrichtung der einzelnen Magnetspulen 14 gleichsinnig. Das Magnetfeld ist gepulst angelegt und wirkt wohltuend auf den Benutzer 11. Es kann zweckmäßig sein, in einem alternativen Ausführungsbeispiel eine andere Anzahl an Magnetspulen 14 vorzusehen, wobei mindestens zwei Magnetspulen 14 vorgesehen sind. Vorzugsweise wird die magnetische Flussdichte des Magnetfeldes von 1000µT nicht überschritten.

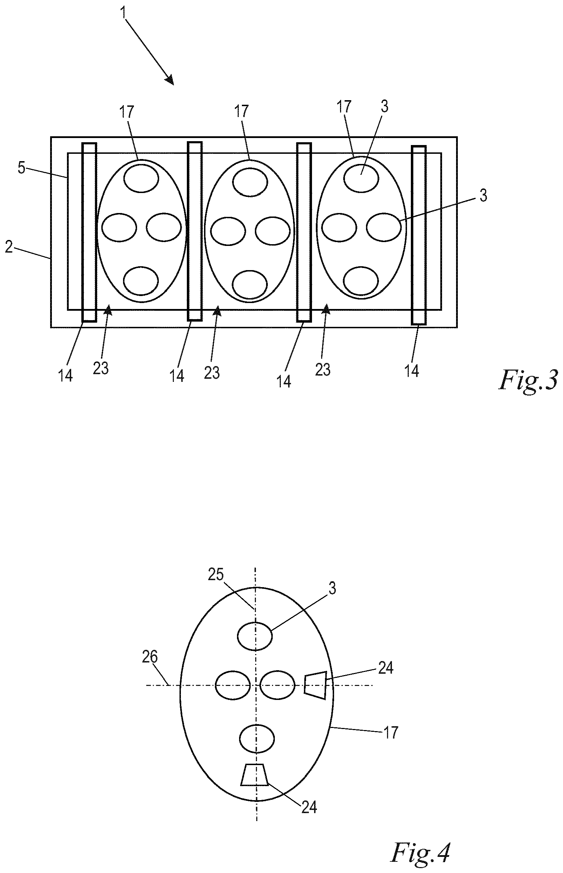

In Fig. 3 ist eine Anordnung mehrerer Infrarotstrahler 3 gezeigt. Zueinander benachbarte Magnetspulen 14 begrenzen einen funktionalen Zwischenraum 23. In dem bevorzugten Ausführungsbeispiel ist in jedem Zwischenraum 23 eine Infrarotstrahler-Einheit 17 angeordnet. Gemäß dem Ausführungsbeispiel umfasst die Wellnessvorrichtung 1 drei Infrarotstrahler-Einheiten 17. Die Infrarotstrahler-Einheit 17 ist aus vier Infrarotstrahlern 3 gebildet. In einer alternativen Ausführungsform der erfindungsgemäßen Wellnessvorrichtung 1 kann es zweckmäßig sein, eine andere Anzahl an InfrarotStrahlern 3 je Infrarotstrahler-Einheit 17 vorzusehen. Vorzugsweise umfasst die Wellnessvorrichtung 1 höchstens 20 Infrarotstrahler 3. Die Infrarotstrahler 3 sind an einem nicht dargestellten Montagerahmen oberhalb des Behandlungsraumes 5 angeordnet. Wie in Fig. 4 gezeigt, sind die Infrarotstrahler 3 über Schrittmotoren 24 um eine erste Schwenkachse 25 und um eine senkrecht zur ersten Schwenkachse 25 angeordnete zweite Schwenkachse 26 schwenkbar angetrieben. Zudem kann es in einer alternativen Ausführungsform der Wellnessvorrichtung 1 zweckmäßig sein, eine Bewegungseinheit in der Wellnessvorrichtung 1 vorzusehen, welche beispielsweise eine lineare, kreisförmige oder ähnliche Bewegungsform der Infrarotstrahler 3 vorsieht. Während des Betriebs der Wellnessvorrichtung 1 werden die Infrarotstrahler 3 permanent über die Schrittmotoren 24 bewegt, wodurch eine andauernde, lokale Bestrahlung des Benutzers 11 sowie eine lokale Überhitzung vermieden wird. Über die Steuereinheit 18 können die Infrarotstrahler 3 nach den spezifischen Anforderungen des Benutzers 11 während des Betriebes der Wellnessvorrichtung 1 eingestellt und bewegt werden.

Wie in den Figuren 1 und 2 gezeigt, umfasst die Wellnessvorrichtung 1 Ultraschallsensoren 16. Die Ultraschallsensoren 16 sind im Behandlungsraum 5, vorzugsweise benachbart zum B/C-Absorber, angeordnet. Die Ultraschallsensoren 16 sind mit der Steuereinheit 18 verbunden und dienen zur Regelung des Abstandes zwischen Benutzer 11 und den Infrarotstrahlern 3. Die Ultraschallsensoren 16 messen permanent den Abstand zum Benutzer 11. Ist der Abstand zu gering, wird dieser über die Steuereinheit 18 der Wellnessvorrichtung 1 eingeregelt. Dabei wird die Höhe der Liegefläche 6 mittels der Hubverstellvorrichtung 12 auf die entsprechende Höhe verfahren. Überhitzungen an dem Benutzer 11 werden dadurch vermieden.

Wie in den Figuren 1 und 2 gezeigt, sind in dem Behandlungsraum 5 UVC-Strahler 10 angeordnet. Ist die Wellnessbehandlung abgeschlossen, kann durch die Aktivierung der UVC-Strahler 10 eine Desinfektion des Behandlungsraums 5 erfolgen. Die Liegefläche 6 umfasst eine aus dem Behandlungsraum 5 herausragende Kopfstütze 27. Die Kopfstütze 27 wird vor der Desinfektion des Behandlungsraumes 5 derart umgeklappt, dass der Behandlungsraum 5 geschlossen ist, und die Desinfektion erfolgen kann. Vorzugsweise umfasst die Wellnessvorrichtung 1 nicht dargestellte, bauliche Elemente, welche ein Öffnen des Gehäuses 2 während einer UVC-Behandlung des Behandlungsraumes 5 verhindern.

Bei Benutzung der Wellnessvorrichtung 1 wird der Benutzer durch die Infrarotstrahler mit A-Infrarotstrahlung bestrahlt und zeitgleich einem homogenen, gepulsten Magnetfeld ausgesetzt. Die Steuereinheit 18 regelt die Wellnessvorrichtung 1 derart, dass die Basalttemperatur, also die Kerntemperatur des Benutzers 11, eine maximale Temperatur in Höhe von 38,5° nicht überschreitet. Die Basalttemperatur des Benutzers ist beispielsweise mittels eines Körpertemperaturmessgerätes zu messen. Die am Stück vorgesehene Dauer der Wellnessbehandlung beträgt vorzugsweise weniger als eine Stunde, insbesondere weniger als 40 min, vorteilhaft in etwa 30 min. Das Magnetfeld wie auch die Infrarotstrahlung entfalten eine wohltuende Wirkung auf den Benutzer. Die Wirkung der Magnetfeldbehandlung erfolgt frei von elektrischer Stimulation.

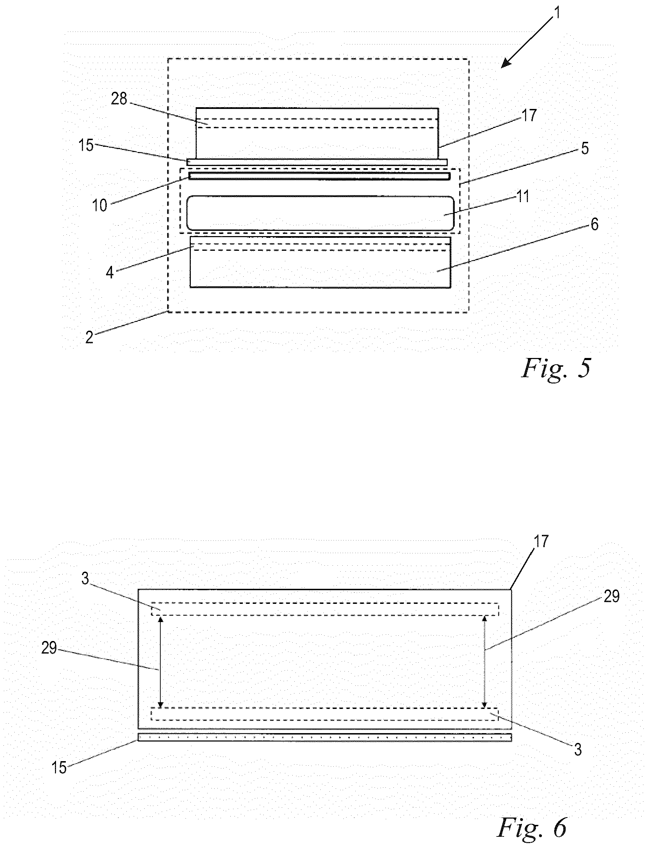

Die Figuren 5 bis 9 zeigen ein erfindungsgemäßes, alternatives Ausführungsbeispiel der Wellnessvorrichtung 1. In der Fig. 5 ist eine Vorderansicht der Wellnessvorrichtung 1 gezeigt. Die Infrarotstrahler-Einheit 17 ist an einem mit dem Gehäuse 2 verbundenen Montagerahmen 28 befestigt. Unterhalb der Infrarotstrahler-Einheit 17 ist der B/C-Absorber angeordnet. Demnach ist der B/C-Absorber der Infrarotstrahler-Einheit 17 nachgeschaltet. Unterhalb des B/C-Absorbers ist der Behandlungsraum 5 angeordnet. Zur Behandlung des Benutzers 11 kann dieser auf der Liegefläche 6 im Behandlungsraum 5 positioniert werden. Zudem ist im Behandlungsraum 5 mindestens ein UCV-Strahler zur Desinfektion des Behandlungsraumes 5 angeordnet. Ferner umfasst die Wellnessvorrichtung 1 eine Magnetfeldeinheit 4 zur Erzeugung eines homogenen Magnetfeldes.

In Fig. 6 ist eine Höhenverstellung der Infrarotstrahler 3 gezeigt. Entsprechend der obigen Ausführungen wurde bereits dargestellt, dass es notwendig ist, den Abstand zwischen dem Benutzer 11 und den Infrarotstrahlern 3 einstellen zu können. Während das erste Ausführungsbeispiel nach Fig. 1 eine höhenverstellbare Liegefläche 6 vorsieht, wird im Ausführungsbeispiel nach Fig. 6 der Abstand zwischen Benutzer 11 und den Infrarotstrahlern 3 über eine Höhenverstellung der Infrarotstrahler 3 selbst eingestellt. Die Pfeile 29 stellen dabei schematisch die Verstellbarkeit der Infrarotstrahler 3 dar.

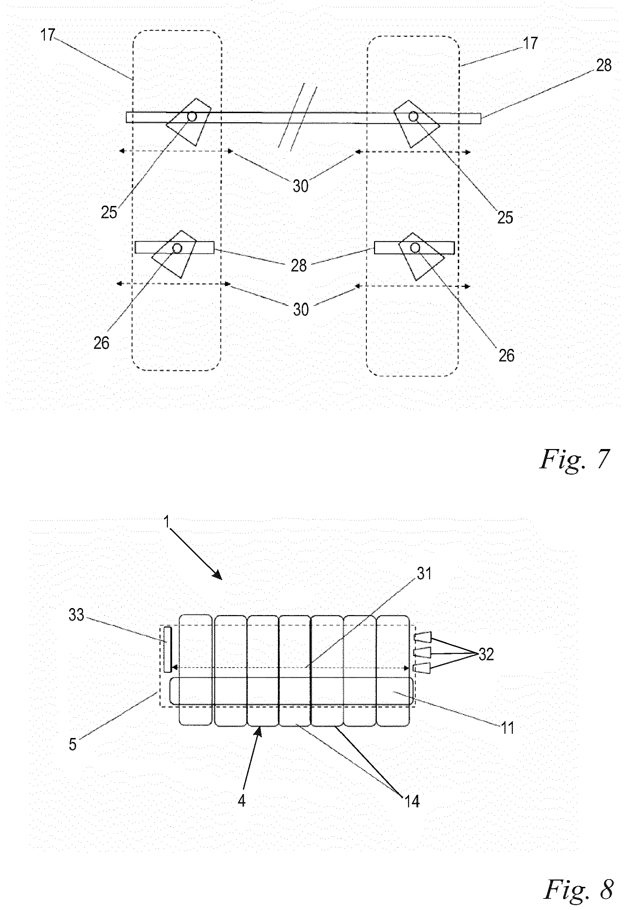

In Fig. 7 ist eine Verstelleinheit der Infrarotstrahler-Einheiten 17 gezeigt. Die Infrarotstrahler-Einheiten 17 sind an dem Montagerahmen 28 angeordnet. Die Infrarotstrahler-Einheiten 17 sind schwenkbar um die erste Schwenkachse 25 und die zweite Schwenkachse 26 gelagert. Zudem sind die Infrarotstrahler-Einheiten 17 in einer Verfahrrichtung 30, die der Längsrichtung des Gehäuses 2 entspricht, verfahrbar. Es kann zweckmäßig sein, andere Richtungen zum Verfahren der Infrarotstrahler-Einheiten 17 vorzusehen. Wie bereits oben ausführlich beschrieben, werden die Infrarotstrahler-Einheiten während des Betriebes der Wellnessvorrichtung 1 bedarfsgerecht bewegt, um Überhitzungen an Hautstellen des Benutzers 11 zu vermeiden.

In Fig. 8 ist die Wellnessvorrichtung 1 in einer Seitenansicht gezeigt. In Längsrichtung der Wellnessvorrichtung 1 sind mehrere Magnetspulen 14 angeordnet, die die Magnetfeldeinheit 4 bilden. Die Magnetspulen 14 umschließen den Behandlungsraum 5 ringartig. Im Behandlungsraum 5 sind zudem an einem Ende Lichtschranken 32 und an dem anderen Ende des Behandlungsraumes ein Lichtreflektor 33 angeordnet. Der Pfeil 31 stellt dabei schematisch einen Laserstrahl da, der von der Lichtschranke 32 gesendet wird und am Reflektor zurückgespiegelt wird. Anhand der Lichtschranken 32 kann der Abstand des Benutzers 11 zu den Infrarotstrahl-Einheiten 17 erkannt werden. Befindet sich der Benutzer 11 also in einem Laserstrahl 31 einer Lichtschranke 32, werden die Infrarotstrahler-Einheiten 17 nach oben bewegt, um den Abstand zum Benutzer 11 wieder zu erhöhen. Dadurch können unerwünschte Erwärmungen des Benutzers 11 vermieden werden.

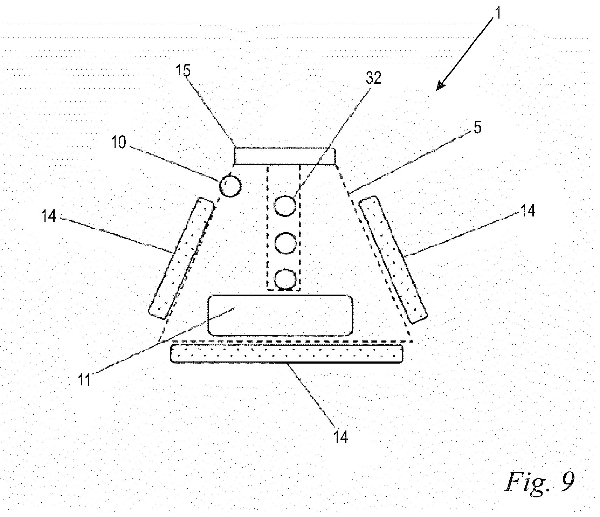

In Fig. 9 ist die Wellnessvorrichtung nach Fig. 5 in einer schematischen Vorderansicht gezeigt. Dabei ist die Anordnung der Magnetspulen 14 zu erkennen. Die drei Magnetspulen 14 sind trigonometrisch, in etwa ein Dreieck bildend, angeordnet. Die Magnetspulen 14 sind außerhalb des Behandlungsraumes 5 angeordnet. Eine Magnetspule 14 ist an der Unterseite, und jeweils eine Magnetspule 14 an den Außenseiten des Behandlungsraumes 5 angeordnet. Die trigonometrische Anordnung ermöglicht die Erzeugung eines homogenen Magnetfeldes etwa in der Mitte des Behandlungsraumes 5.

Bei dem erfindungsgemäßen Verfahren sind vorzugsweise eine kurzwellige Infrarot Wärmebehandlung mit einer gepulsten Magnetfeld Behandlung in einem Gerät kombiniert. Vorteilhaft kann eine dreidimensionale Positionierung eines einzelnen Temperaturstrahlers sensorgesteuert durchgeführt werden. Vorzugsweise ist in dem Verfahren eine dreidimensionale Positionierung von bis 20 Temperaturstrahlern gleichzeitig und sensorgesteuert automatisch durchführbar. Vorteilhaft wird eine dreidimensionale Positionierung von bis zu 20 Temperaturstrahlern verwendet, um eine voreingestellte Wärmedosis lokal nicht zu überschreiten. Es ist vorzugsweise vorgesehen, dass ein Infrarot-Absorber nur mittel- und langwelliges Infrarot bei der Passage durch eine dünne Salzwasserschicht absorbiert. Vorzugsweise verändert ein Infrarot-Absorber für nicht absorbiertes Infrarot-A die Winkel der Lichtbrechung durch ein flüssiges Medium ständig. Es ist vorteilhaft vorgesehen, dass ein Infrarot-Absorber für nicht absorbiertes Infrarot-A die Winkel der Lichtbrechung durch ein flüssiges Medium so verändert, dass die Dosis von kurzwelligem Infrarot nicht konstant auf einem lokalen Bereich verbleibt. Nach jeder durchgeführten Therapie wird vorzugsweise der benutzte Behandlungsraum im Gerät durch UVC-Strahlenmit einer Wellenlänge von 253,7nm desinfiziert.

In einem alternativen Ausführungsbeispiel sind vorteilhaft mehrere Magnetfelder gleichzeitig aktiv und vorzugsweise mindestens drei Magnetspulen in einem Modul trigonometrisch zusammengefasst. Es ist vorzugsweise vorgesehen, dass mindestens drei Magnetspulen in trigonometrischer Anordnung so zusammengefasst werden, dass eine geschlossene magnetische Ringspule nachgebildet wird.

## Patentansprüche

1. Wellnessvorrichtung,
umfassend ein Gehäuse (2), wobei das Gehäuse (2) einen sich in Längsrichtung des Gehäuses (2) erstreckenden Behandlungsraum (5) umschließt, und mit einer zentralen Steuereinheit (18) zur Steuerung der Wellnessvorrichtung (1), **dadurch gekennzeichnet, dass** an dem Gehäuse (2) mindestens ein Infrarotstrahler (3) zur Erzeugung von Infrarotstrahlung im Behandlungsraum (5) und eine Magnetfeldeinheit (4) zur Erzeugung eines im Wesentlichen homogenen Magnetfeldes im Behandlungsraum (5) angeordnet sind.

2. Wellnessvorrichtung nach Anspruch 1,
**dadurch gekennzeichnet, dass** die Magnetfeldeinheit (4) Magnetspulen (14) umfasst, wobei die Magnetspulen (14) in einer Helmholtzanordnung angeordnet sind.

3. Wellnessvorrichtung nach Anspruch 2,
**dadurch gekennzeichnet, dass** die Magnetspulen (14) ringartig ausgebildet sind und den Behandlungsraum (5) umschließen.

4. Wellnessvorrichtung nach einem der Ansprüche 1 bis 3,
**dadurch gekennzeichnet, dass** dem Infrarotstrahler (3) ein B/C-Absorber (15) zur Filterung von mittel- und langwelliger Infrarotstrahlung nachgeschaltet ist.

5. Wellnessvorrichtung nach Anspruch 4,
**dadurch gekennzeichnet, dass** der B/C-Absorber (15) separat von dem mindestens einen Infrarotstrahler (3) ausgebildet ist.

6. Wellnessvorrichtung nach Anspruch 4 oder 5,
**dadurch gekennzeichnet, dass** der B/C-Absorber (15) zwei zueinander parallel angeordnete Glasplatten (20) umfasst, und die Glasplatten (20) einen Hohlraum (21) einschließen, wobei der Hohlraum (21) mit Salzwasserlösung (22) gefüllt ist.

7. Wellnessvorrichtung nach Anspruch 6,
**dadurch gekennzeichnet, dass** der Abstand der Glasplatten (20) mindestens 1 mm beträgt.

8. Wellnessvorrichtung nach einem der Ansprüche 1 bis 7,
**dadurch gekennzeichnet, dass** der mindestens eine Infrarotstrahler (3) um eine erste Schwenkachse (25) schwenkbar ist.

9. Wellnessvorrichtung nach Anspruch 8,
**dadurch gekennzeichnet, dass** der mindestens eine Infrarotstrahler (3) um eine zweite Schwenkachse (26) schwenkbar ist

10. Wellnessvorrichtung nach Anspruch 8 oder 9,
**dadurch gekennzeichnet, dass** der mindestens eine Infrarotstrahler (3) zum Schwenken durch einen über die zentrale Steuereinheit (18) gesteuerten Schrittmotor (24) für jede Schwenkachse (25, 26) angetrieben ist.

11. Wellnessvorrichtung nach einem der Ansprüche 2 bis 10,
**dadurch gekennzeichnet, dass** zwei zueinander benachbarte Magnetspulen (14) einen funktionalen Zwischenraum (23) begrenzen, wobei der mindestens eine Infrarotstrahler (3) in dem funktionalen Zwischenraum (23) angeordnet ist.

12. Wellnessvorrichtung nach einem der Ansprüche 1 bis 11,
**dadurch gekennzeichnet, dass** die Vorrichtung mindestens einen UVC-Strahler (10) zur Desinfektion des Behandlungsraumes (5) umfasst.

13. Wellnessvorrichtung nach einem der Ansprüche 1 bis 12,
**dadurch gekennzeichnet, dass** das erzeugte Magnetfeld gepulst ist.

14. Verfahren mit einer Wellnessvorrichtung nach den Ansprüchen 1 bis 13 umfassend die folgenden Schritte:
Zeitgleiche Erzeugung von Infrarotstrahlung und eines im Wesentlichen homogenen Magnetfeldes im Behandlungsraum des Gehäuses (2).

15. Verfahren nach Anspruch 14,
**dadurch gekennzeichnet, dass** der Benutzer (11) im Behandlungsraum (5) auf eine maximale Temperatur von höchstens 38,5°C erwärmt wird.
